# EUROPEAN PATENT APPLICATION

(11) **EP 3 892 237 A1**
(43) Date of publication of application: **13.10.2021**
(21) Application number: 20706049.2
(22) Date of filing: 25.02.2020
(51) Int. Cl.: A61F 2/12, A61F 2/00, A61B 17/34, A61B 17/00

(54) **IMPLANT TRANSFER DEVICE**

(71) Applicant: KIMS-MED CO., LTD., Buk-gu, Gwangju 61003 (KR)
(72) Inventor: KIM, Song Hee, Anyang-si, Gyeonggi-do 14102 (KR)
(74) Representative: Isarpatent
(86) International application number: PCT/KR2020/002707
(87) International publication number: WO 2021/172612

(57) **Abstract**

The present invention provides an implant delivery device. The present invention includes a sleeve configured to deliver an implant through the inside thereof, wherein the sleeve includes a body tapered from one end to the other end, and fine irregularities disposed on an inner surface of the body to reduce a frictional force between the body and the implant.

## Description

### [Technical Field]

The present invention relates to a device, and more specifically, to an implant delivery device which delivers an implant inserted into the human body.

### [Background Art]

Surgical methods are continuously evolving, and a constant theme of this development is the desire to reduce incision sizes and potential for infection, such that the body suffers less trauma in surgery, reduced post-operative scarring, and secondary operations. This is a particular issue in the area of breast augmentation and reconstruction, where the long-term appearance of the breast is an important consideration in determining whether the operation has been successful.

Many patients prefer pre-filled breast implants (such as silicone implants), as they can offer a more natural appearance than implants that are filled in situ. However, pre-filled implants are inevitably larger and require larger incisions, resulting in the need to develop delivery devices that can place the pre-filled breast implant in the surgical pocket through smaller and smaller incisions, without damage to the implant. One solution to this is the apparatus developed and sold by Keller Medical Inc. as the Keller Funnel@ (described in US 8,211,173). The Keller funnel is a tapered sleeve which is used in the manner of an icing bag, the breast implant being introduced into a larger end of the sleeve, and extruded into the surgical pocket through a smaller end of the sleeve.

However, while Keller sleeves have been very successful, it remains possible for the breast implant to become damaged or contaminated prior to delivery to the surgical pocket, because of the need to handle the breast implant during transfer from the sterile packaging it is received in, to the sleeve. Contamination is generally regarded as being linked to infection and capsular contracture, and to avoid this standard surgical practice is to change gloves immediately prior to handling the implant. To further reduce the likelihood of contamination, it has been suggested that pre-packaging a breast implant in a Keller Funnel@ could be advantageous (US 2015/0032208). However, pre-packaging in this way may not reduce the handling required, as there is a chance that the implant would fall out of the sleeve during transit and storage. In addition, if the sleeve required activation prior to use, for instance with water or a saline solution, the implant may need to be removed from the sleeve to facilitate this. Further, packing a Keller Funnel@ with a breast implant can complicate the initial product sterilization process, as commercially available Keller Funnels® cannot be sterilized using the dry heat sterilization methods typically used to sterilize implants. This is because the funnel is designed to deform when heated to prevent reuse. As such, the packaging of a Keller Funnel@ directly with a breast implant would require either the redesign of the funnel, or adoption of an alternative sterilization process to the cost effective dry heat sterilization generally used for implant sterilization.

### [Disclosure]

### [Technical Problem]

The present invention is directed to providing an implant delivery device capable of easily delivering an implant. Further, the present invention is directed to providing an implant delivery device capable of preventing contamination due to contact. However, the above are exemplary, and the present invention is not limited thereto.

### [Technical Solution]

One aspect of the present invention provides an implant delivery device including a sleeve configured to deliver an implant through the inside thereof, wherein the sleeve includes a body tapered from one end to the other end, and fine irregularities disposed on an inner surface of the body to reduce a frictional force between the body and the implant.

The fine irregularities may be disposed at a portion where friction between the body and the implant occurs.

The fine irregularities may be disposed at a portion adjacent to the other end of the body.

Each of the fine irregularities may include a hemispherical end portion.

The implant delivery device may include a first end region which seals the other end of the body.

The body may include a guide line to cut the first end region according to a size of the implant.

The implant delivery device may further include a second end region which is located at one end of the body and is opened and closed.

Aspects, features, and advantages other than the above will become apparent by specific contents, claims, and drawings for the invention which will be described later.

### [Advantageous Effects]

An implant delivery device according to one embodiment of the present invention can have various functions. The implant delivery device can more smoothly deliver the implant.

An implant delivery device according to one embodiment of the present invention can prevent contamination due to contact. Of course, the present invention is not limited by the above.

### [Description of Drawings]

FIG. 1 is a view schematically illustrating an implant delivery device according to one embodiment of the present invention.
FIG. 2 is a cross-sectional view schematically illustrating a cross-section taken along line II-II in FIG. 1.
FIG. 3 is a modification of the implant delivery device according to FIG. 2.
FIG. 4 is a view schematically illustrating an implant delivery device according to another embodiment of the present invention.
FIGS. 5 to 10 are use state views of the implant delivery device according to the embodiment of the present invention.

### [Modes of the Invention]

Here, various embodiments of the disclosure are disclosed in connection with the accompanying drawings. Since the various embodiments of the disclosure may be variously changed and have various embodiments, particular embodiments are exemplified in the drawings and related detailed descriptions are disclosed. However, the various embodiments of the disclosure are not limited to the particular embodiments and include all changes, equivalents, and substitutes within the spirit and the scope of the present invention. In the description of the drawings, similar reference numerals are used for similar elements.

Terms "include," "may include," and the like usable in in the various embodiments of the disclosure indicate the presence of functions, operations, elements, or the like which are disclosed and do not limit one or more additional functions, operations, elements, or the like. Further, in the various embodiments of the disclosure, it should be further understood that the terms "include," "including," "provide," "providing," "have," and/or "having" specify the presence of stated features, integers, steps, operations, elements, components, and/or groups thereof but do not preclude the presence or addition of one or more other features, integers, steps, operations, elements, components, and/or groups thereof.

In the various embodiments of the disclosure, terms "or" and the like include any, and all combinations of terms listed together. For example, "A or B" may include A, B, or both A and B.

Terms "first," "second," "primary", "secondary," or the like used in the various embodiments of the disclosure may modify various elements of the various embodiments, but do not limit the elements. For example, the terms do not limit the order or importance of the elements. The terms may be used to distinguish one element from another. For example, both a first user device and a second user device are user devices, and refer to different user devices. For example, a first element could be termed a second element, and similarly, a second element could be termed a first element without departing from the scope of the various embodiments of the disclosure.

When a certain component is mentioned as being "linked," "coupled," or "connected" to another component, the component may be directly linked or connected to the other component, but it should be understood that additional components may be present therebetween. On the other hand, when the certain component is mentioned as being "directly linked," "directly coupled," or "directly connected" to the other component, it should be understood that no additional components are present between the certain component and the other component.

Terms used the various embodiments of the disclosure are used just to describe the particular embodiments, and not to limit the various embodiments of the disclosure. The singular form is intended to also include the plural form, unless the context clearly indicates otherwise.

Unless otherwise defined, all terms including technical or scientific terms used in the present invention have meanings the same as those of terms generally understood by those skilled in the art.

It will be further understood that terms, such as those defined in commonly used dictionaries, should be interpreted as having a meaning that is consistent with their meaning in the context of the relevant art and will not be interpreted in an idealized or overly formal sense unless expressly so defined herein.

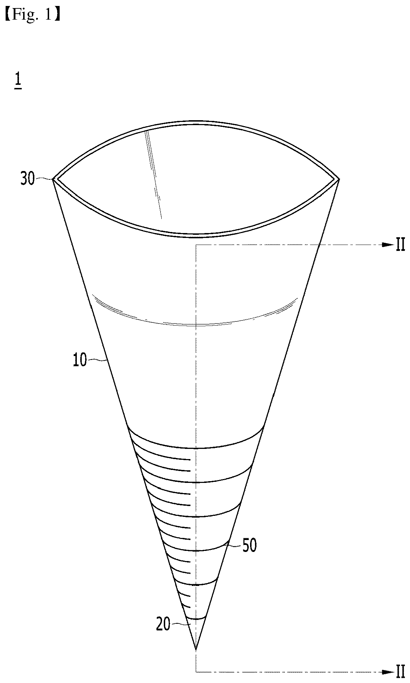

FIG. 1 is a view schematically illustrating an implant delivery device according to one embodiment of the present invention. The implant delivery device according to the embodiment includes a sleeve 1. The sleeve 1 may deliver the implant 2 to a surgical pocket. The implant 2 is delivered to the surgical pocket of a patient through the inside of the sleeve 1. The sleeve 1 includes a cavity therein. Here, the implant 2 may be a breast implant 2. Further, the surgical pocket refers to an incised portion of an armpit or chest of the patient.

More specifically, the sleeve 1 is tapered from one end to the other end. For example, the sleeve 1 may have a conical shape. Of course, the present invention is not limited thereto, and a portion of the sleeve 1 may be formed in a cylindrical shape, and the remaining portion of the sleeve 1 may be formed in a conical shape. The implant 2 may be introduced into the one end of the sleeve 1 and discharged at the other end. Alternatively, the sleeve 1 may have a pyramid shape. As described above, the sleeve 1 may be variously modified.

In this case, in the sleeve 1, the other end which is pointed is sealed as described below. In order to insert the implant 2 into the surgical pocket, the other end of the sleeve 1 is cut during surgery.

The sleeve 1 includes a flexible material. That is, the sleeve 1 is formed of a soft material such as vinyl or a thin fabric, which may be easily crumpled. For example, the sleeve 1 may be formed of polyvinyl chloride (PVC), polyethylene terephthalate (PET), or the like.

Accordingly, when the surgeon applies pressure to the sleeve 1, the implant 2 receives the pressure, and may be moved to the surgical pocket. Further, the implant 2 may easily slide along an inner side surface of the sleeve 1.

The sleeve 1 includes a transparent material. As described above, since the sleeve 1 includes a transparent material, the surgeon may directly observe a state of the implant 2 inserted into the sleeve 1 with the naked eye. That is, the surgeon may observe a moving degree and moving direction of the implant 2, and thus may control a moving speed and moving direction of the implant 2 by adjusting the pressure. Further, the surgeon may pressurize the implant 2 by adjusting the pressure so that the implant 2 is not damaged while the pressure is applied.

The sleeve 1 includes a body 10 and a first end region 20. The sleeve 1 may further include a second end region 30.

The body 10 is tapered from one end to the other end. Further, the body 10 includes a cavity therein. The implant 2 may be introduced into and discharged from the cavity of the body 10. For example, the body 10 may have a conical shape. Of course, the present invention is not limited thereto, and the body 10 may be variously modified like the above-described sleeve 1.

The first end region 20 is located at the other end of the body 10. For example, the first end region 20 refers to a narrow portion of a funnel. Further, the first end region 20 seals the other end of the body 10. That is, as described above, the sleeve 1 includes the funnel in which the first end region 20 is sealed. The first end region 20 may be removed later to introduce the implant 2.

Meanwhile, the second end region 30 is disposed to be spaced apart from the first end region 20. The second end region 30 may be located at one end portion of the body 10. For example, as shown in the drawing, the second end region 30 may be located at a circular portion (an upper portion which is shown) of the conical body 10. As another example, the second end region 30 may be located at an upper portion of the side portion of the conical body 10.

In this case, the second end region 30 is open so that the implant 2 may be introduced. Alternatively, both the second end region 30 and the first end region 20 may be sealed by a zipper, an adhesive, a tape, or the like, or may be sealed by a clip in an overlapping state.

The implant 2 is introduced into the body 10 through the second end region 30, and is discharged through a portion from which the first end region 20 is removed.

Meanwhile, an inner surface of the sleeve 1 (or the body 10) may be coated with a hydrophilic coating.

It is advantageous if the inner surface of the tapered sleeve has a low friction coefficient, as this allows the breast implant to pass more easily through the delivery device. The low friction coefficient may be achieved through the selection of a material for the delivery device which inherently has a low friction coefficient, through treatment of the inner surface of the sleeve to reduce the friction coefficient, or through the provision of a lubricant. Treatment of the inner surface of the sleeve could be through the application of a hydrophilic coating, which on contact with water generates a lubricious surface, or the application of a hydrophobic coating, which would remove the need for hydration. The hydrophilic coating may be applied using a range of techniques, often, application will be through spray coating or on-lay of the coating.

It can be advantageous for the inner surface of the tapered sleeve to be lubricated as this is an inexpensive option, allowing the materials used for the body of the sleeve to be less expensive. As noted above, it may be that the lubrication of the inner surface of the tapered sleeve is through the use of a substance which is lubricious, or a coating which becomes lubricious when activated through contact with water or a saline solution. Where contact with water or a saline solution is required, activation will be a step prior to delivery of the implant. Typically, the water or saline solution will be applied to the inner surface of the sleeve after opening, however, it is possible that water or a saline solution will be present in the packaging with the implant. The lubricant will most often be an organic or synthetic oil, such as silicone oil or KY Jelly (propylene glycol combined with hydroxypropylmethyl cellulose (HPMC)). Lubricants which may be used in the subject invention include, ComfortCoat™ (DSM), VitroStealth™ (DSM), Harmony™ (SurMedics), Baymedix™ CL100 (Bayer), Hydak™ T070 (BioCoat), iSurGlide™ (ISurTec) and Lubrilast™ (AST). These hydrophilic lubricants often comprise one or more of polyvinyl pyrrolidone, polyvinyl alcohols, propylene oxide, polyethylene oxide, polyacrylamides, polyvinyl ethers, hyaluronic acid, or combinations thereof.

The lubricant may also be a cellulose-based lubricant, such as a lubricant containing HMPC, or carboxymethyl cellulose. Further, polyelectrolytes may be used, such as salts of homo- and copolymers of acrylic acid, methacrylic acid, maleic acid, sulfonic acid, quaternary ammonium salts and combinations of all of the above.

In order to activate the hydrophilic coating, a solution L is introduced into the body 10. For example, the surgeon introduces the solution L into the sleeve 1, and activates the hydrophilic coating by grasping and shaking both ends of the sleeve 1. In this case, since the surgeon comes into contact with both end regions of the sleeve 1, the sleeve 1 which comes into contact with a surgical portion may be contaminated.

However, since the first end region 20 of the implant delivery device according to the embodiment is blocked, the surgeon introduces the solution L into the body 10 through the second end region 30. Further, the surgeon may activate the hydrophilic coating by shaking the sleeve 1 without coming into contact with the first end region 20.

Accordingly, the implant delivery device according to the embodiment may significantly reduce the possibility of contamination of the first end region 20 and a portion adjacent to the first end region 20. Even when the surgeon touches the first end region 20, the first end region 20 is minimally touched compared to the conventional case, and the first end region 20 which comes into contact with the surgeon is removed later to significantly reduce the possibility of contamination.

An outlet 11 from which the implant 2 is discharged is formed by cutting the first end region 20. The sleeve 1 includes a guide line 50 showing a size of the outlet 11 according to the size of the implant 2 on an outer surface thereof. Accordingly, the surgeon may simply and accurately cut the first end region 20 by adjusting an opening degree of the outlet 11 according to the size of the implant 2.

Each of the plurality of guide lines 50 has a marker which shows the size of the implant 2. The implant 2 may have a marker corresponding to the guide line 50.

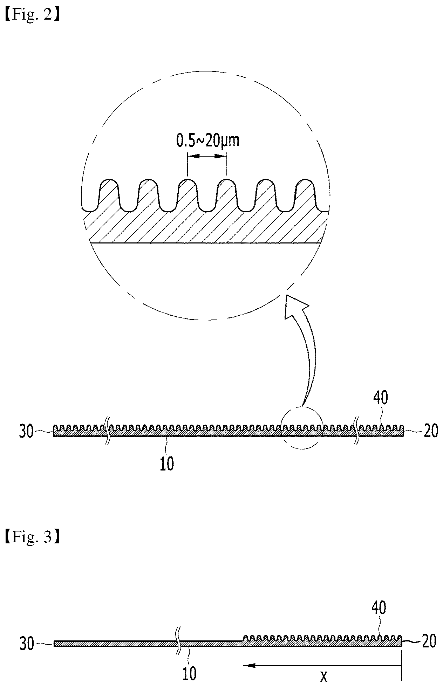

FIG. 2 is a cross-sectional view schematically illustrating a cross-section unevenness taken along line II-II in FIG. 1. A cross-section of the sleeve 1 will be described in more detail with reference to FIG. 2 according to the embodiment.

An inner surface of the body 10 includes fine irregularities 40 which reduce a frictional force with the implant 2. When pressure is applied to the implant 2, it is difficult for the implant 2 to slide due to the frictional force with the surface of the body 10. The inner surface of the body 10 may be treated to reduce the frictional force to smoothly introduce the implant 2 into the surgical pocket. In this case, the sleeve 1 may include or not include the hydrophilic coating on the inner surface. Fine irregularities 40 can also be called micro unevenness or micro protrusion.

More specifically, the inner surface of the body 10 includes the fine irregularities 40 which reduce the frictional force with the implant 2. That is, the inner surface of the body 10 may have a micro texture formed therein or include an undulating surface. The fine irregularities 40 reduce a contact area between the surface of the body 10 and the implant 2, to reduce the frictional force generated between the body 10 and the implant 2. In this case, the fine irregularities 40 may be disposed throughout an entire inner surface of the body 10.

The fine irregularity 40 may be spaced apart from the surface of the body 10 at an interval of roughly 0.5 to 20 um and may be formed in plural. Of course, the present invention is not limited thereto, and the fine irregularities 40 may be formed at an interval capable of reducing the frictional force.

Each of the fine irregularities 40 include a hemispherical end portion. For example, as shown in the drawing, an upper portion of each of the fine irregularities 40 is formed in a hemispherical shape to prevent the implant 2 from being damaged. When the implant 2 is damaged by the fine irregularities 40, bacteria or foreign matter may settle in a damaged portion, and cause cancer. The fine irregularities 40 may prevent this problem.

Here, the upper portion of each of the fine irregularities 40 refers to a portion which is spaced far apart from the surface of the body 10, and comes into contact with the implant 2.

Referring to FIG. 3, the fine irregularities 40 may be disposed at a portion where the friction between the body 10 and the implant 2 occurs. That is, the fine irregularities 40 may be partially formed in the inner surface of the body 10, and specifically, may be disposed around the outlet 11 through which the implant 2 exits. The outlet 11 is formed in the other end of the body 10. Here, the outlet 11 may be formed by cutting the first end region 20.

The sleeve 1 has been described as having the outlet 11 sealed by the first end region 20 but is not limited thereto, and the outlet 11 may be formed in advance in the sleeve 1. That is, the sleeve 1 may include one end and the other end, which are open.

Accordingly, productivity may be improved, costs may be reduced, and a manufacturing process may be simplified by forming the fine irregularities 40 in only necessary portions.

Specifically, the fine irregularities 40 may be disposed at a portion adjacent to the first end region 20. The fine irregularities 40 may be formed up to a predetermined distance x in a direction toward the one end from the other end of the body 10. Here, the other end portion of the body 10 may be an end of the first end region 20, or an end portion of the body 10 after the first end region 20 is cut.

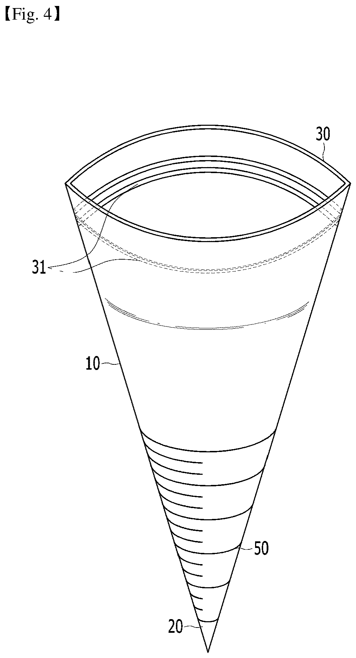

Referring to FIG. 4, the implant delivery device further includes the second end region 30 which may be opened and closed. Here, the second end region 30 is an inlet into which the implant 2 is introduced. Here, the second end region 30 further includes an opening and closing unit. The opening and closing unit may be reused.

For example, the opening and closing unit may be a sealing zipper 31 which is provided in the inner surface of the body 10, and is reusable. A portion of the opening and closing unit is provided in one surface of the body 10, and the other portion is provided in the other surface of the body 10. The portion and the other portion of the opening and closing unit correspond to each other, and may be coupled and separated.

Further, the opening and closing unit is elongated in a width direction of the body 10, and the portion and the other portion abut each other at both end portions. Accordingly, the opening and closing unit may tightly seal the second end region 30.

Here, a longitudinal direction of the body 10 refers to a direction from one end to the other end, and the width direction is a direction approximately perpendicular to the longitudinal direction.

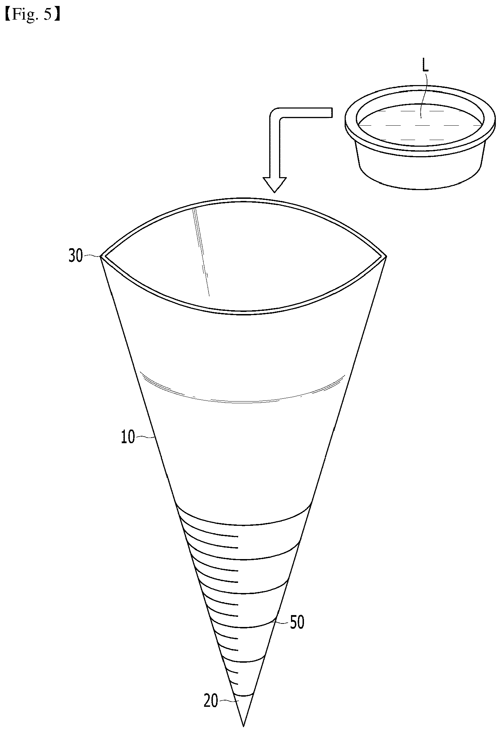

FIGS. 5 to 10 are views schematically illustrating a method of using the implant delivery device. Here, a method of using the implant delivery device according to the above-described embodiment of FIG. 1 will be described.

Referring to FIG. 5, an operation of opening the sleeve 1 is carried out to open the sleeve 1. The sleeve 1 is delivered to the surgeon in a compressed state, and thus is delivered in the form of a flat triangle. Accordingly, the surgeon forms an empty space in the sleeve 1 to input the implant 2 and/or the solution L into the sleeve 1.

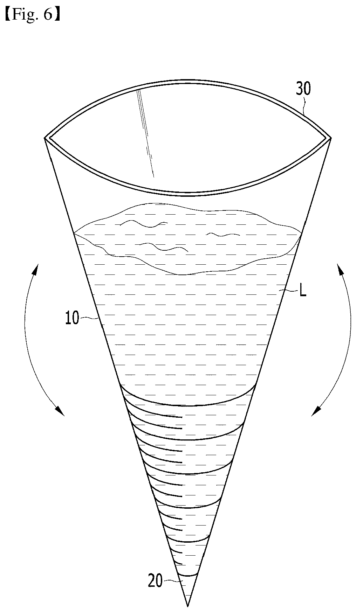

Referring to FIG. 6, an operation of activating the hydrophilic coating is performed. In the operation of activating the hydrophilic coating, the solution L evenly activates the hydrophilic coating lubricating the inner surface of the body 10.

For example, since the first end region 20 is blocked, the surgeon inputs the solution L into the sleeve 1 through the second end region 30. Further, the surgeon activates the hydrophilic coating by holding the second end region 30 and/or a region adjacent thereto and shaking the sleeve 1 without touching the first end region 20. The surgeon may block or not block the second end region 30 when shaking the sleeve 1.

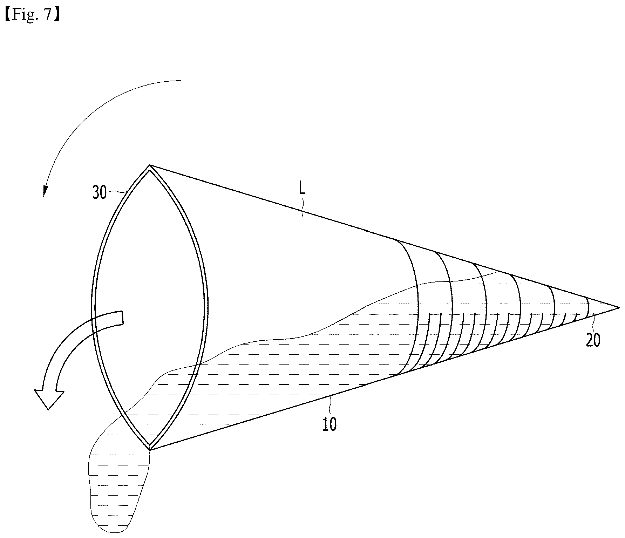

Referring to FIG. 7, the surgeon may pour the solution L from the sleeve 1 after activating the hydrophilic coating. In this case, the surgeon may pour the solution L from the sleeve 1 through the second end region 30. Alternatively, after removing the first end region 20 which will be described later, the surgeon may pour the solution L from the sleeve 1 through the first end region 20.

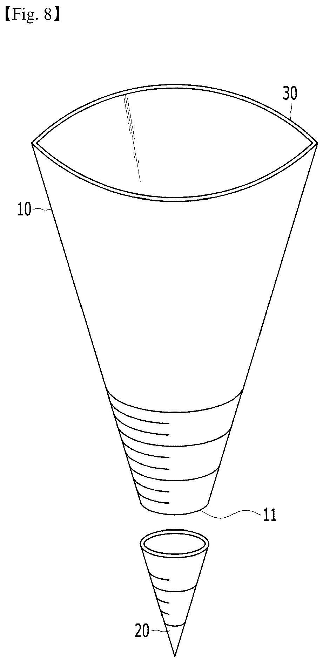

Referring to FIG. 8, an operation of removing the first end region 20 which forms the outlet 11 in the sleeve 1 by removing the first end region 20 is performed. The surgeon removes the first end region 20 along the guide line 50 corresponding to the size of the implant 2.

The surgeon may hold the first end region 20 and cut the first end region 20 along the guide line 50. In this case, since the first end region 20 touched by the surgeon is removed, the body 10 of the sleeve 1 may not be contaminated by contact.

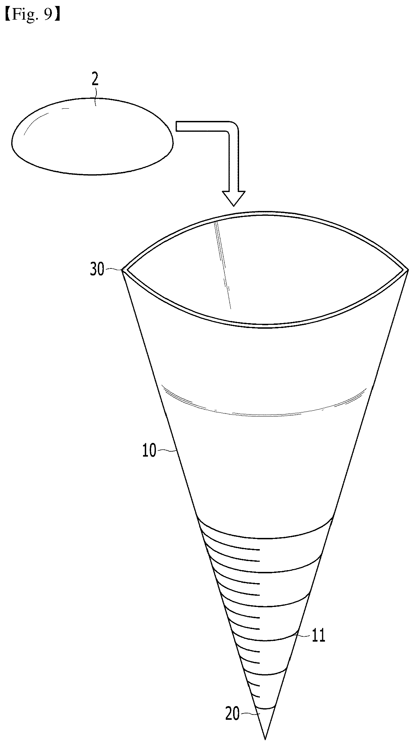

Referring to FIG. 9, the surgeon may input the implant 2 into the sleeve 1 and then remove the first end region 20. Alternatively, the surgeon may input the implant 2 into the sleeve 1 after removing the first end region 20.

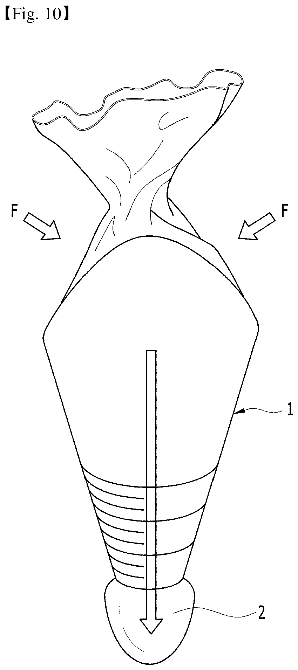

Referring to FIG. 10, further, the surgeon inputs the outlet 11 of the sleeve 1 into the surgical pocket, and manually applies pressure to the sleeve 1 to move the implant 2 into the surgical pocket.

A method of delivering the implant 2 using the implant delivery device according to the embodiment may significantly reduce the possibility of contamination of the first end region 20 and a portion adjacent to the first end region 20. Even when the surgeon touches the first end region 20, the first end region 20 is minimally touched compared to the conventional case, and the first end region 20 which comes into contact with the surgeon is removed later to significantly reduce the possibility of the contamination.

As described above, the present invention has been described with reference to embodiments shown in the drawings but these are only exemplary, and it may be understood by those skilled in the art that various modifications and other equivalents are possible therefrom. Accordingly, the technical scope of the present invention should be determined by the technical spirit of the appended claims.

### [Reference numerals]

- 1:: sleeve
- 10:: body
- 20:: first end region
- 30:: second end region
- 40:: fine irregularities
- 50:: guide line

## Claims

1. An implant delivery device comprising a sleeve configured to deliver an implant through the inside thereof,
wherein the sleeve includes a body tapered from one end to the other end, and fine irregularities disposed on an inner surface of the body to reduce a frictional force between the body and the implant.

2. The implant delivery device of claim 1, wherein the fine irregularities are disposed at a portion where friction between the body and the implant occurs.

3. The implant delivery device of claim 2, wherein the fine irregularities are disposed at a portion adjacent to the other end of the body.

4. The implant delivery device of claim 1, wherein each of the fine irregularities includes a hemispherical end portion.

5. The implant delivery device of claim 1, comprising a first end region which seals the other end of the body.

6. The implant delivery device of claim 5, wherein the body includes a guide line to cut the first end region according to a size of the implant.

7. The implant delivery device of claim 1, further comprising a second end region which is located at one end of the body and is opened and closed.
